(19) Europäisches Patentamt
European Patent Office
Office européen des brevets

(11) **EP 2 230 258 A1**

(12) **EUROPÄISCHE PATENTANMELDUNG**

(43) Veröffentlichungstag:
**22.09.2010 Patentblatt 2010/38**

(51) Int Cl.:
*C08B 37/14* *(2006.01)*     *C08G 73/02* *(2006.01)*
*A61K 31/205* *(2006.01)*

(21) Anmeldenummer: **09450152.5**

(22) Anmeldetag: **25.08.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **18.03.2009 EP 09450060**

(71) Anmelder: **Mindinvest Holdings Ltd.**
**1122 Valleta VLT, MT (MT)**

(72) Erfinder: **Schmidt, Oskar**
**1030 Wien (AT)**

(74) Vertreter: **Schwarz, Albin**
**Schwarz & Partner**
**Patentanwälte**
**Wipplingerstraße 30**
**1010 Wien (AT)**

(54) **Mikrobiozid wirkendes Arzneimittel**

(57) Arzneimittel, enthaltend ein in fester Form vorliegendes, mikrobiozid wirkendes Polymergemisch, welches ein erstes Polymer als Matrix aufweist, in welche ein zweites Polymer eingebracht ist, wobei das zweite Polymer ein polymeres Guanidin- oder Biguanidinderivat ist.

**EP 2 230 258 A1**

**Beschreibung**

Die vorliegende Erfindung betrifft ein mikrobiozid wirkendes Arzneimittel.

Mikrobiozid wirkende Polymere auf der Basis von Guanidinium-Hydrochlorid, insbesondere deren Wirkung gegen *Escherichia* coli-Bakterien, sind bereits bekannt (vgl. WO 01/85676). Weiterhin ist bereits bekannt, dass solche Guanidin-Derivate als fungizide Mittel verwendet werden können (vgl. WO 2006/047800). Von besonderer Bedeutung sind die Polymere Akacid®, das Poly-[2-(2-ethoxy)-ethoxyethyl-guanidiniumchlorid], und Akacid plus®, eine 3:1-Mischung aus Poly-(hexamethylenguanidiniumchlorid) und Poly-[2-(2-ethoxy)-ethoxyethyl)-guanidiniumchlorid] (vgl. Antibiotika Monitor, 22. Jahrgang, Heft 1/2/2006, Online-Ausgabe unter http://www.antibiotikamonitor.at/ 06_12/06_12_inhalt.htm).

Die genannten mikrobizid wirkenden Polymere gehören zur Gruppe der kationischen Antiseptika, welche chemisch sehr unterschiedliche Substanzen umfassen, die aber als gemeinsames Charakteristikum stark basische Gruppen, gebunden an ein ziemlich massives lipophiles Molekül, besitzen. Die wichtigsten Vertreter sind unter den Quarternären Ammoniumverbindungen Benzalkoniumchlorid und Cetrimid, unter den Bisbiguaniden Chlorhexidin und Alexidin und unter den polymerischen Biguaniden Polyhexamethylen-Biguanid (PHMB).

Aufgrund ihrer eigenen positiv geladenen Moleküle haben die kationisch antimikrobiell wirksamen Substanzen eine hohe Bindungsaffinität an die negativ geladenen Zellwände und Membranen von Bakterien. Durch Störung dieser Angriffspunkte kommt es zunächst zur Herabsetzung der Membranfluidität und zu einer Störung der osmoregulatorischen und physiologischen Zellfunktionen. In weiterer Folge entstehen hydrophile Poren in der Phospholipidmembran, und die Proteinfunktion wird gestört. Das Endresultat ist eine Lyse der Zielzelle. Dieser Membran-schädigende Wirkmechanismus konnte auch für die polymerischen Guanidine gegenüber *Escherichia coli* demonstriert werden.

Aus der WO 99/54291 sind Polyhexamethylenguanidine bekannt, die aufgrund ihrer mikrobioziden Wirkung als Desinfektionsmittel eingesetzt werden können. Diese Stoffe werden durch Polykondensation von Guanidin mit einem Alkylendiamin, insbesondere Hexamethylendiamin, hergestellt. Das erhaltenene Kondensationsprodukt besitzt eine gute biozide Wirkung.

Aus der WO 2006/047800 A1 ist ein polymeres Kondensationsprodukt bekannt, das durch Umsetzen von Guanidin oder seinem Salz mit einem Alkylendiamin und einem Oxyalkylendiamin erhalten werden kann. Dieses Kondensationsprodukt wirkt biozid und insbesondere fungizid. Ein Vertreter dieses Kondensationsprodukts ist als auch als "Akacid Plus" im Handel.

Aus der WO 2008/080184 A2 wiederum ist bekannt die Herstellung und Verwendung von polymeren Guanidinium-Hydroxiden auf Basis eines Diamins, welches Oxyalkylenketten und/oder Alkylengruppen zwischen zwei Aminogruppen enthält und erhältlich ist durch Polykondensation eines Guanidin-Säureadditionssalzes mit dem Diamin, wobei ein Polykondensationsprodukt in Salzform erhalten wird, welches anschließend durch basischen Anionenaustausch in die Hydroxidform überführt wird, zur Bekämpfung von Mikroorganismen.

Im Stand der Technik sind ferner Arzneimittel bekannt, welche die polymeren Guanidinderivate als Wirkstoffe enthalten, antimikrobiell wirken und in der Humanmedizin, wie auch in der Veterinärmedizin zur Bekämpfung von Infektionen eingesetzt werden können.

Es hat sich bei der Anwendung der Arzneimittel, z.B. im Veterinärbereich, gezeigt, dass Geflügel Trinkwasser verweigerte, dem das polymere Guanidinderivat zugesetzt worden war. Ähnliches wurde bei der Verfütterung an Schweinen beobachtet, also wenn granulatförmiges polymeres Guanidinderivat dem Schweinefutter beigemischt wurde.

Die vorliegende Erfindung stellt sich daher als Aufgabe, polymere Guanidine zur Verfügung zu stellen, welche die oben genannten Nachteile nicht aufweisen oder in einem verringerten Maß aufweisen. Aufgabe der Erfindung ist somit, den therapeutischen Anwendungsbereich der polymeren Guanidin- und Biguanidinderivaten zu vergrößern.

Diese Aufgabe wird gelöst, indem die mikrobizid wirkenden polymeren Guanidine in ein Polymer eingebracht werden. Es hat sich gezeigt, dass diese Polymer als Matrix wirken, aus der die Guanidine offenbar nicht nennenswert herausgewaschen werden können. Aus diesem Grund kommt es nicht zu den erwähnten nachteiligen Effekten. Das Überraschende dabei ist aber, dass die mikrobizide Wirkung trotzdem erhalten bleibt.

Die Erfindung betrifft daher ein Arzneimittel, welches ein in fester Form vorliegendes, mikrobiozid wirkendes Polymergemisch enthält, welches ein erstes Polymer als Matrix aufweist, in welche ein zweites Polymer eingebracht ist, wobei das zweite Polymer ein polymeres Guanidin- oder Biguanidinderivat ist.

Im Stand der Technik ist ein Acrylat für Kunststoff-Zahnfüllungen bekannt, welches ein polymeres Guanidin enthält (Österreichisches Forschungsinstitut für Chemie und Technik). Dieses Acrylat besitzt eine Plaque-hemmende Wirkung.

Das polymere Biguanidinderivat kann ein Poly(hexamethylen)biguanid sein.

Das polymere Guanidinderivat ist bevorzugt auf Basis eines Alkylendiamins und/oder eines Oxyalkylendiamins und enthält das Alkylendiamin und das Oxyalkylendiamin im Molverhältnis zwischen 4:1 und 1:4.

Eine weitere bevorzugte Ausfühumgsform des erfindungsgemäßen Arzneimittels ist dadurch gekennzeichnet, dass das mikrobiozid wirkende Polymergemisch als Pulver oder als Granulat ausgebildet ist.

Die mittlere Teilchengröße des erfindungsgemäßen Pulvers liegt bevorzugt im Bereich von 10 nm und 1.000 $\mu$m, insbesondere im Bereich von 50 $\mu$m und 100 $\mu$m, und die mittlere Teilchengröße des Granulats liegt bevorzugt im Bereich von 1.000 $\mu$m und 5,0 mm.

Die Aminogruppen des Alkylendiamins und/oder des Oxyalkylendiamins sind bevorzugt endständig.

Zur Herstellung des polymeren Guanidinderivates ist ferner bevorzugt als Alkylendiamin eine Verbindung der allgemeinen Formel

$$NH_2(CH_2)_nNH_2$$

vorgesehen, in welcher n eine ganze Zahl zwischen 2 und 10, insbesondere 6, ist.

Zur Herstellung des polymeren Guanidinderivates ist darüber hinaus bevorzugt als Oxyalkylendiamin eine Verbindung der allgemeinen Formel

$$NH_2[(CH_2)_2O)]_n(CH_2)_2NH_2$$

vorgesehen, in welcher n eine ganze Zahl zwischen 2 und 5, insbesondere 2, ist.

Eine weitere bevorzugte Ausführungsform des erfindungsgemäßen Arzneimittels besteht darin, dass als polymeres Guanidinderivat ein Polyoxyalkylen-Guanidin auf Basis von Triethylenglykoldiamin (relative Molekularmasse: 148), von Polyoxypropylendiamin (relative Molekularmasse: 230) und/oder von Polyoxyethylendiamin (relative Molekularmasse: 600) vorgesehen ist.

Als polymeres Guanidinderivat eignet sich Poly-[2-(2-ethoxy-ethoxyethyl)-guanidinium-hydrochlorid] mit mindestens 3 Guanidinresten besonders gut.

Die mittlere Molekularmasse des polymeren Guanidinderivates liegt insbesondere im Bereich 500 bis 5.000.

Das polymere Guanidinderivat kann im ersten Polymer in einer Menge zwischen 0,1 Gew.-% und 20 Gew.-%, bezogen auf die Gesamtmasse, enthalten sein.

Das erste Polymer kann ein Thermoplast, ein Duroplast oder ein Elastomer sein. Als härtende Kunststoffe kommen die Phenoplaste, wie Phenol, Resorcin, Kresol, Xylenol-Harz, sowie die Aminoplaste, wie Melamin- und Harnstoff-Harz, in Frage. Der Wirkstoff (das polymere Guanidin) wird nach der Vorkondensation("Novolak") dem Härter (Hexamethylentetramin)zugesetzt.

Ungesättigte Polyesterharze und ölmodifizierte Polyesterharze werden mit dem Härter vermischt, wobei der Härter vorzugsweise ein Peroxyd ist und der Wirkstoff dem Härter zugesetzt wird.

Epoxyharze werden u.a. mit Polyalkohol-Phenol-Amid oder -Amin gehärtet, wobei der Wirkstoff jeweils dem Härter zugesetzt wird.

Bei Polyurethanharz wird der Wirkstoff der Polyol-Komponente zugesetzt.

Bei Polymethylmethacrylat-Harz wird der Wirkstoff ebenfalls dem Härter zugesetzt.

Die Erfindung ist nicht auf die angeführten Beispiele von härtenden Kunststoffen beschränkt. Bei der Auswahl handelt es sich um eine bevorzugte Ausführungsform. Die so erhaltenen Duroplasten werden zerkleinert und feinst vermahlen. Das so gewonnene erfindungsgemäße Pulver kann - je nach Anwendungsgebiet - bei der Herstellung von antimikrobiell wirkenden Medien diesen zugesetzt werden.

Eine weitere bevorzugte Ausführungsform des erfindungsgemäßen Arzneimittels ist ferner dadurch gekenneichnet, dass das Polymergemisch einen weiteren Zusatzstoff, inbesondere $BaSO_4$, enthält. Mit dem Einbringen des spezifisch schweren $BaSO_4$ in die Kunststoffmatrix kann das spezifische Gewicht des erfindungsgemäßen Arzneimittels dem des Wassers angeglichen werden, mit der Folge, dass ein derartiges Pulver im Wasser suspendiert werden kann und in Schwebe bleibt, sich also nicht absetzt. Dies ist z.B. für die Verabreichung an Geflügel von Bedeutung, wo Arzneimittel gerne dem Trinkwasser beigemischt werden.

Das erfindungsgemäße Arzneimittel kann sowohl in der Humanmedizin als auch in der Veterinärmedizin angewendet werden. Besonders bevorzugt ist die Verwendung des in fester Form vorliegenden, mikrobiozid wirkenden Polymergemisches, wie es oben definiert ist, zur Herstellung eines Arzneimittels zur Behandlung von Infektionen des Gastrointestinaltraktes in der Humanmedizin als auch in der Veterinärmedizin.

Ein Vorteil des erfindungsgemäßen Arzneimittels besteht darin, dass der Wirkstoff in der Polymermatrix fest eingebunden ist und nicht herausgelöst wird. Er kann daher auch nicht resorbiert werden, sondern wird bei oraler Verabreichung zusammen mit dem Polymer aus dem Gatrointestinaltrakt mit der Faeces wieder ausgeschieden.

Mit den nachfolgenden Beispielen wird die Erfindung noch näher beschrieben, wobei mit den Beispielen 1 bis 6 die Herstellung von mikrobiozid wirkenden Polymergemischen beschrieben wird. Das Beispiel 7 zeigt die antimikrobielle Wirkung des Polymergemisches, und das Beispiel 8 zeigt die Verwendung in der Veterinärmedizin.

Beispiel 1

Thermoplastisches Polyurethan (Tecoflex TX 1, Fa. Thermomodics) wird in einen gleichläufigen Doppelschnekkenextruder (Typ: Thermo Prism TSE 24 HC) in Zone 3 (von insgesamt 10 thermisch erfassbaren Zonen) eingebracht. Das Polyurethan dient als Matrix für das nachfolgend einzubringende polymere Guanidinderivat.

Als polymeres Guanidinderivat wird das Polykondensat aus 1 Mol Guanidiniumhydrochlorid und einem Gemisch aus 0,75 Mol Hexamethylendiamin und 0,25 Mol Triethylenglykolamin verwendet. Dieses Copolymer ist im Handel erhältlich ("Akacid Plus" der Fa. AKA Technology). Die Herstellung ist in der WO 06/047800 A1 beschrieben. Das Guanidinderivat wird als 75%-ige wässerige Lösung der Zone Nr.5 des Doppelschneckenextruders zudosiert. Das Guanidinderivat wird in einer Menge zudosiert werden, dass sein Anteil im fertigen Gemisch mit dem Polyurethan 2 Gew.-%, bezogen auf die Gesamtmasse, beträgt. Danach wird eine zweite Charge hergestellt, deren gehalt an Guanidinderivat jedoch 4 Gew.-% beträgt.

Die Temperaturverteilung in den einzelnen Zonen des Extruders ist wie folgt:

Zone 1 : 40 ° C
Zone 2 : 170 ° C
Zone 3 : 210 ° C
Zone 4 : 210 ° C
Zone 5 : 190 ° C
Zone 6 : 110 ° C
Zone 7 : 110 ° C
Zone 8 : 190 ° C
Zone 9 : 190 ° C
Zone 10 : 180 ° C
Düse: 180° C

Durch die Extrusion werden das Polyurethan und das polymere Guanidinderivat innig miteinander vermischt. Das extrudierte Gemisch wird durch ein Wasserbad gezogen und anschließend granuliert.

Das Granulat wird zu Pulver mit einer mittleren Teilchengröße von 75 $\mu$m vermahlen. Die Vermahlung kann in kryogenen Feinmühlen erfolgen.

Beispiel 2

Granulatförmiges Hochdruck-Polyäthylen (Typ: Borstar MB6561, Fa.Borealis, spezifisches Gewicht 0,96), als Matrix, Schwerspat ($BaSO_4$, spezifisches Gewicht 4,48), 4%, und pulverförmiges Poly-hexamethylendiaminguanidiniumchlorid, das "Akacid Forte" der Firma AKA Technology, 10 %, wird in einen gleichläufigen Doppelschneckenextruder (Typ: Thermo Prism TSE 24 HC) in Zone 3 (von insgesamt 10 thermisch erfassbaren Zonen) eingebracht. Die Temperaturverteilung in den einzelnen Zonen ist wie folgt:

Zone 1 : 40 ° C
Zone 2 : 150 ° C
Zone 3 : 180 ° C
Zone 4 : 180 ° C
Zone 5 : 180 ° C
Zone 6 : 160 ° C
Zone 7 : 160 ° C
Zone 8 : 190 ° C
Zone 9 : 190 ° C
Zone 10 : 190 ° C
Düse: 170° C

Das Gemisch wird extrudiert, durch ein Wasserbad gezogen und anschließend granuliert. Dieses Granulat wird analog zu Beispiel 1 zu einem Pulver weiterverarbeitet.

Dieses Pulver wird mit Wasser in einer Kugelmühle zu einer Suspension verarbeitet, welche der Tränke bzw. den Getränken als Anti-Infektivum zugesetzt wird.

Beispiel 3

Als Matrix wird Polyamid 6 (Typ: Fürkamid B,SK1, Fa.Solvadis Polym.) in einen gleichläufigen Doppelschnek-kenextruder (Typ: Thermo Prism TSE 24 HC) in Zone 3 (von insgesamt 10 thermisch erfassbaren Zonen) eingebracht. In der Zone Nr.5 werden 5% des wie Beispiel 1 angegebenen Guanidincopolymers "Akacid Plus" der Fa. AKA Technology zugesetzt.

Die Temperaturverteilung in den einzelnen Zonen ist wie folgt:

Zone 1 : 40 ° C

Zone 2 : 220 ° C

Zone 3 : 240 ° C

Zone 4 : 250 ° C

Zone 5 : 230 ° C

Zone 6 : 140 ° C

Zone 7 : 140 ° C

Zone 8 : 240 ° C

Zone 9 : 240 ° C

Zone 10 : 230 ° C

Düse: 230° C

Das Gemisch wird extrudiert, durch ein Wasserbad gezogen und anschließend granuliert. Dieses Granulat wird analog zu Beispiel 1 zu einem Pulver weiterverarbeitet.

Beispiel 4

Als Matrix wird Polypropylen ( Typ: PPH7062, Fa.Total Chemicals) in einen gleichläufigen Doppelschneckenextruder ( Typ: Thermo Prism TSE 24 HC) in Zone 3 ( von insgesamt 10 thermisch erfassbaren Zonen) eingebracht. In der Zone Nr.5 wird eine 75%ige wässerige Lösung des in Beispiel 1 angegebenen Guanidincopolymers "Akacid Plus" der Fa. AKA Technology zudosiert. Die Wirkstoffkonzentration beträgt in diesem Fall 5% des Gemisches.

Die Temperaturverteilung in den einzelnen Zonen ist wie folgt:

Zone 1 : 40 ° C

Zone 2 : 150 ° C

Zone 3 : 150 ° C

Zone 4 : 160 ° C

Zone 5 : 160 ° C

Zone 6 : 160 ° C

Zone 7 : 160 ° C

Zone 8 : 170 ° C

Zone 9 : 180 ° C

Zone 10 : 180 ° C

Düse: 170° C

Das Gemisch wird extrudiert, durch ein Wasserbad gezogen und anschließend granuliert. Dieses Granulat wird analog zu Beispiel 1 zu einem Pulver weiterverarbeitet.

Beispiel 5

Als Matrix wird Polycarbonat ( Typ: MakrolonRX1805, Fa.Bayer ) in einen gleichläufigen Doppelschneckenextruder ( Typ: Thermo Prism TSE 24 HC) in Zone 3 ( von insgesamt 10 thermisch erfassbaren Zonen) eingebracht. In der Zone Nr.5 wird eine 75%ige wässerige Lösung des in Beispiel 1 angegebenen Guanidincopolymers, "Akacid Plus" der Fa. AKA Technology, zudosiert.
Die Temperaturverteilung in den einzelnen Zonen ist wie folgt:

Zone 1 : 40 ° C
Zone 2 : 150 ° C
Zone 3 : 240 ° C
Zone 4 : 250 ° C
Zone 5 : 230 ° C
Zone 6 : 140 ° C
Zone 7 : 140 ° C
Zone 8 : 240 ° C
Zone 9 : 240 ° C
Zone 10 : 230 ° C
Düse: 230° C

Das Gemisch wird extrudiert, durch ein Wasserbad gezogen und anschließend granuliert. Dieses Granulat wird analog zu Beispiel 1 zu einem Pulver weiterverarbeitet.
Die Wirkstoffkonzentration beträgt in diesem Fall 4% des Gemisches.

Beispiel 6

Als Matrix wird Acryl-Butadien-Styrol (Typ: PA727, ein thermoplastisches ABS der Fa.Chi-Mei,Taiwan) in einen gleichläufigen Doppelschneckenextruder ( Typ: Thermo Prism TSE 24 HC) in Zone 3 (von insgesamt 10 thermisch erfassbaren Zonen) eingebracht. In der Zone Nr.5 wird eine 75%ige wässerige Lösung des in Beispiel 1 angegebenen Guanidincopolymers "Akacid Plus" der Fa. AKA Technology zudosiert.
Die Temperaturverteilung in den einzelnen Zonen ist wie folgt:

Zone 1 : 40 ° C

Zone 2 : 170 ° C

Zone 3 : 200 ° C

Zone 4 : 200 ° C

Zone 5 : 180 ° C

Zone 6 : 110 ° C

Zone 7 : 110 ° C

Zone 8 : 190 ° C

Zone 9 : 190 ° C

Zone 10 : 180 ° C

Düse: 180° C

Das Gemisch wird extrudiert, durch ein Wasserbad gezogen und anschließend granuliert. Dieses Granulat wird analog zu Beispiel 1 zu einem Pulver weiterverarbeitet.

Die Wirkstoffkonzentration bewegt sich in diesem Fall zwischen 10% und 20%, vorzugsweise 20%.

Beispiel 7

Qualtitative Untersuchung der bakteriziden Wirkung des erfindungsgemäß als Wirkstoff eingesetzten Polymergemisches mit Hilfe des Japanischen Industrie Standards JIS Z 2801 (2000)

Die Prüfung der Oberflächenwirksamkeit erfolgt gemäß Verfahren JIS Z 2801:2000 ("Antimicrobial products-Test for antimicrobial activity and efficacy") mit den Testkeimen *Escherichia coli* (ATCC 10536) oder *Staphylococcus aureus* (ATCC 6538). Hierfür werden 2cm x 3cm oder 5cm x 5cm große Testflächen mit einer 0,2 ml Keimsuspension entsprechender Keimdichte beimpft und mit PE-Folie abgedeckt. Die Inkubation erfolgt bei einer Luftfeuchtigkeit > 90% und einer Temperatur von 35°C über 24 Stunden. Danach werden die Bakterien mit physiologischer Kochsalzlösung von den Testflächen abgewaschen und die Lebenskeimzahlen nach 24 oder 48 Stunden ermittelt. Die Berechnung der Reduktionszahlen (R-Werte) erfolgt nach folgender Formel:

$$R = [\log (B/A) - \log (C/A)]$$

R-Wert: Wert der antimikrobiellen Aktivität, Reduktionszahl
A-Wert: Lebendkeimzahl am wirkstofffreien Vergleichsmuster direkt nach dem Beimpfen
B-Wert: Lebendkeimzahl am wirkstofffreien Vergleichsmuster nach 24h Inkubation
C-Wert: Lebendkeimzahl am wirkstoffhaltigen Teststück nach 24-48h Inkubation

Als antimikrobiell aktiv werden alle die Stoffe angesehen, die zu einer Log-Reduktion von zwei oder mehr führen.

Für den Test wurden die beiden in Beispiel 1 beschriebenen Polymergemische mit einem Gehalt an dem polymeren Guanidin von 2 Gew.-% bzw. 4 Gew.-% verwendet. Die Ergebnisse sind in der nachfolgenden Tabelle 1 angegeben.

Tabelle 1 Wirkung gegen *E. coli* und *S. aureus.*

| Tecoflex, 2% polym. Guanidin | E. coli |
|---|---|
| A-Wert [KBE/Probe] | 1,20E+07 |
| B-Wert [KBE/Probe] | 1,36E+07 |
| C-Wert [KBE/Probe] mit 2% | 3,37E+05 |
| R-Wert | > 2 |
| Tecoflex, 4% polym. Guanidin | E. coli |
| A-Wert [KBE/Probe] | 1,20E+07 |
| B-Wert [KBE/Probe] | 1,36E+05 |
| C-Wert [KBE/Probe] mit 4% | 7,80E+05 |
| R-Wert | > 1 |
|  |  |
|  |  |
| Tecoflex, 2% polym. Guanidin | S. aureus |
| A-Wert [KBE/Probe] | 1,00E+05 |
| B-Wert [KBE/Probe] | 1,06E+07 |

(fortgesetzt)

| Tecoflex, 2% polym. Guanidin | S. aureus |
|---|---|
| C-Wert [KBE/Probe] mit 2% | 1,01E+05 |
| R-Wert | > 2 |
| | |
| Tecoflex, 4% polym. Guanidin | S. aureus |
| A-Wert [KBE/Probe] | 1,00E+05 |
| B-Wert [KBE/Probe] | 1,06E+07 |
| C-Wert [KBE/Probe] mit 4% | 4,20E+04 |
| R-Wert | > 2 |

Beispiel 8

Es wurde gemäß Beispiel 2 ein Pulver hergestellt. Dieses Pulver wurde mit Reinstwasser in einer Kugelmühle im Verhältnis 1:1 vermahlen. Die so erhaltene Suspension wurde dem Trinkwasser 1:50 zugesetzt und den Schweinen, welche mit E.coli K 88 infiziert wurden, verabreicht.

Die Behandlungsdauer betrug 14 Tage. Zum Vergleich diente eine Behandlung mit dem Breitbandantibiotikum Amoxycillin. Das Ergebnis ist der nachfolgenden Tabelle 2 zu entnehmen. Sie zeigt die Anzahl der fäkalen E. coli K 88 (als log 10) in Abhängigkeit vom Behandlungstag. Der Ausgangswert (Behandlungstag: 0) betrug $10^6$ Keime. Die Tabelle zeigt für beide Wirkstoffe, dass die Bakterienanzahl zu Beginn der Behandlung zunächst noch ansteigt, wobei das Maximum an den Tagen 4 bzw. 5 erreicht wird, wonach die Bakterienanzahl wieder abnimmt und am Tag 14 den Ausgangswert wieder erreicht. Mit dem erfindungsgemäßen Arzneimittel kann die Bakterienanzahl entscheidend unter dem für Amoxycillin erhaltenen Wert gehalten werden: $10^{7,7}$ für die Erfindung (Tag 4) gegen $10^{8,2}$ für Amoxycillin. Dies zeigt, dass sich das erfindungsgemäße Arzneimittel hervorragend als Antibiotika-Ersatz eignet. Gleich gute Resultate wurden bei Zugabe eines Granulates zum Futter mit einer Teilchengröße von 1.200 μ erreicht.

Tabelle 2

| Behandlungstag | 0 | 2 | 3 | 4 | 5 | 6 | 10 | 14 |
|---|---|---|---|---|---|---|---|---|
| Erfindung | 6,0 | 5,9 | 7,0 | 7,7 | 6,6 | 6,4 | 6,5 | 6,2 |
| Amoxycillin | 6,0 | 7,2 | 7,8 | 7,6 | 8,2 | 7,1 | 8,0 | 6,4 |

**Patentansprüche**

1. Arzneimittel, enthaltend ein in fester Form vorliegendes, mikrobiozid wirkendes Polymergemisch, welches ein erstes Polymer als Matrix aufweist, in welche ein zweites Polymer eingebracht ist, wobei das zweite Polymer ein polymeres Guanidin- oder Biguanidinderivat ist.

2. Arzneimittel nach Anspruch 1, **dadurch gekennzeichnet, dass** das polymere Biguanidinderivat ein Poly(hexamethylen)biguanid ist.

3. Arzneimittel nach Anspruch 1, **dadurch gekennzeichnet, dass** das polymere Guanidinderivat auf Basis eines Alkylendiamins und/oder eines Oxyalkylendiamins ist.

4. Arzneimittel nach einem der Ansprüche 1 bis 3, **dadurch gekennzeichnet, dass** das mikrobiozid wirkende Polymergemisch als Pulver oder als Granulat ausgebildet ist.

5. Arzneimittel nach Anspruch 4, **dadurch gekennzeichnet, dass** die mittlere Teilchengröße des Pulvers im Bereich von 10 nm und 1.000 μm, insbesondere im Bereich von 50 μm und 100 μm, liegt, und dass die mittlere Teichengröße des Granulats im Bereich von 1.000 μm und 5,0 mm liegt.

**6.** Arzneimittel nach einem der Ansprüche 3 bis 5, **dadurch gekennzeichnet, dass** ein polymeres Guanidinderivat vorgesehen ist, welches das Alkylendiamin und das Oxyalkylendiamin im Molverhältnis zwischen 4:1 und 1:4 enthält.

**7.** Arzneimittel nach einem der Ansprüche 3 bis 6, **dadurch gekennzeichnet, daß** die Aminogruppen des Alkylendiamins und/oder des Oxyalkylendiamins endständig sind.

**8.** Arzneimittel nach einem der Ansprüche 3 bis 7, **dadurch gekennzeichnet, daß** zur Herstellung des polymeren Guanidinderivates als Alkylendiamin eine Verbindung der allgemeinen Formel

$$NH_2(CH_2)_nNH_2$$

vorgesehen ist, in welcher n eine ganze Zahl zwischen 2 und 10, insbesondere 6, ist.

**9.** Arzneimittel nach einem der Ansprüche 3 bis 8, **dadurch gekennzeichnet, daß** zur Herstellung des polymeren Guanidinderivates als Oxyalkylendiamin eine Verbindung der allgemeinen Formel

$$NH_2[(CH_2)_2O)]_n(CH_2)_2NH_2$$

vorgesehen ist, in welcher n eine ganze Zahl zwischen 2 und 5, insbesondere 2, ist.

**10.** Arzneimittel nach einem der Ansprüche 3 bis 9, **dadurch gekennzeichnet, dass** als polymeres Guanidinderivat ein Polyoxyalkylen-Guanidin auf Basis von Triethylenglykoldiamin (relative Molekularmasse: 148), von Polyoxypropylendiamin (relative Molekularmasse: 230) und/oder von Polyoxyethylendiamin (relative Molekularmasse: 600) vorgesehen ist.

**11.** Arzneimittel nach einem der Ansprüche 3 bis 10, **dadurch gekennzeichnet, dass** als das polymere Guanidinderivat Poly-[2-(2-ethoxy-ethoxyethyl)-guanidinium-hydrochlorid] mit mindestens 3 Guanidinresten vorgesehen ist.

**12.** Arzneimittel nach einem der Ansprüche 1 bis 11, **dadurch gekennzeichnet, dass** die mittlere Molekularmasse des polymeren Guanidinderivates im Bereich 500 bis 5.000 liegt.

**13.** Arzneimittel nach einem der Ansprüche 1 bis 12, **dadurch gekennzeichnet, dass** das erste Polymer ein Thermoplast, ein Duroplast oder ein Elastomer ist.

**14.** Arzneimittel nach einem der Ansprüche 1 bis 13, **dadurch gekennzeichnet, dass** das zweite Polymer zu 0,1 Gew.-% bis 20 Gew.-% im ersten Polymer enthalten ist.

**15.** Arzneimittel nach einem der Ansprüche 1 bis 12, **dadurch gekenneichnet, dass** das Polymergemisch einen weiteren Zusatzstoff, insbesondere BaSO$_4$, enthält.

**16.** Arzneimittel nach einem der Ansprüche 1 bis 15 zur veterinärmedizinischen Anwendung.

**17.** Verwendung eines in fester Form vorliegendes, mikrobiozid wirkendes Polymergemisches, wie es in einem der Ansprüche 1 bis 15 definiert ist, zur Herstellung eines Arzneimittels zur Behandlung von Infektionen des Gastrointestinaltraktes in der Humanmedizin als auch in der Veterinärmedizin.

Europäisches
Patentamt

European
Patent Office

Office européen
des brevets

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 0 619 272 A (AGFA GEVAERT NV [BE]) 12. Oktober 1994 (1994-10-12) * Ansprüche 1,2 * * Seite 3, Zeile 43 - Zeile 44 * | 1-17 | INV. C08B37/14 C08G73/02 A61K31/205 |
| A,D | EP 0 439 698 A (DEGUSSA [DE]) 7. August 1991 (1991-08-07) * Ansprüche * | 1 | |
| X | EP 1 918 306 A (UNIV NEW BRUNSWICK [CA]) 7. Mai 2008 (2008-05-07) * Ansprüche 1,5 * * Beispiele * | 1-17 | |
| A | US 2007/270552 A1 (ZHENG ANNA [CN] ET AL) 22. November 2007 (2007-11-22) * Ansprüche 14-16 * | 1-17 | |

RECHERCHIERTE SACHGEBIETE (IPC)

C08B
C08G
A61K

Der vorliegende Recherchenbericht wurde für alle Patentansprüche erstellt

| Recherchenort | Abschlußdatum der Recherche | Prüfer |
|---|---|---|
| München | 15. Februar 2010 | West, Nuki |

EP 2 230 258 A1

**ANHANG ZUM EUROPÄISCHEN RECHERCHENBERICHT
ÜBER DIE EUROPÄISCHE PATENTANMELDUNG NR.**

EP 09 45 0152

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-02-2010

| Im Recherchenbericht angeführtes Patentdokument | | Datum der Veröffentlichung | Mitglied(er) der Patentfamilie | | Datum der Veröffentlichung |
|---|---|---|---|---|---|
| EP 0619272 | A | 12-10-1994 | DE | 69317259 D1 | 09-04-1998 |
| | | | DE | 69317259 T2 | 01-10-1998 |
| | | | JP | 2724435 B2 | 09-03-1998 |
| | | | JP | 7000975 A | 06-01-1995 |
| | | | US | 5423990 A | 13-06-1995 |
| EP 0439698 | A | 07-08-1991 | DE | 4002403 A1 | 01-08-1991 |
| EP 1918306 | A | 07-05-2008 | CA | 2608824 A1 | 30-04-2008 |
| | | | US | 2008139441 A1 | 12-06-2008 |
| US 2007270552 | A1 | 22-11-2007 | AT | 400596 T | 15-07-2008 |
| | | | AU | 2003221218 A1 | 29-09-2003 |
| | | | WO | 03078490 A1 | 25-09-2003 |
| | | | CN | 1445270 A | 01-10-2003 |
| | | | DK | 1486519 T3 | 17-11-2008 |
| | | | EP | 1486519 A1 | 15-12-2004 |
| | | | ES | 2311091 T3 | 01-02-2009 |
| | | | JP | 2005520877 T | 14-07-2005 |
| | | | US | 2005133952 A1 | 23-06-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0185676 A
- WO 2006047800 A
- WO 9954291 A

- WO 2006047800 A1
- WO 2008080184 A2
- WO 06047800 A1